# EUROPEAN PATENT SPECIFICATION

(11) **EP 3 994 015 B1**
(45) Date of publication and mention of the grant of the patent: **22.11.2023**
(21) Application number: 20742967.1
(22) Date of filing: 01.07.2020
(51) Int. Cl.: B60H 1/00, A61B 5/18, G01N 33/497, B60K 28/06, A61B 5/08, A61B 5/00

(54) **COMBINED VEHICLE MOUNTED BREATH ANALYZING AND HVAC SYSTEM AND METHOD**
KOMBINIERTES FAHRZEUGMONTIERTES ATEMANALYSE- UND HLK-SYSTEM SOWIE VERFAHREN
SYSTÈME ET PROCÉDÉ D'ANALYSE D'HALEINE ET DE CVC COMBINÉS MONTÉS SUR UN VÉHICULE

(30) Priority: 03.07.2019 SE 1950840
(43) Date of publication of application: 11.05.2022
(73) Proprietor: Senseair AB, 820 60 Delsbo (SE)
(72) Inventor: LJUNGBLAD, Jonas, 112 56 Stockholm (SE)
(74) Representative: Zacco Sweden AB
(86) International application number: PCT/SE2020/050690
(87) International publication number: WO 2021/002796

(56) References cited:
- DE-A1-102012 202 649
- JP-A- 2009 226 992
- KR-A- 20180 120 834

## Description

### Field of the invention

The present invention relates to a breath analyzing system and method. In particular the invention relates to a breath analyzing system combined with a heat, ventilation and air conditioning system (HVAC-system) of the vehicle, wherein the settings of the HVAC system are controlled to optimize the performance of the breath analyzing system.

### Background of the invention

Breath analyzing equipment for analyzing occurrences and concentration of intoxicating substances in a person's breath is becoming increasingly common, not at least in vehicles as a measure to detect and prevent driving under the influence of intoxicating substances. The main focus is ethanol (ethyl alcohol/drinking alcohol) detection, but also other substances are of interest. The breath analyzing equipment mounted in vehicles typically gives a measured value of the content of a substance or substances in the driver's breath, which in turn, to a high accuracy, relates to the concentration in the blood of the user. The breath analyzing equipment may be part of a system also including equipment for identifying the driver and/or immobilizing the vehicle, so called "alcolocks". Such breath analyzing equipment is typically permanently mounted in the vehicle and may be an integral part of the dashboard, for example, and connected to the control system of the vehicle. Up to date, such systems have been found primarily in commercial vehicles like buses, taxis and trains. However, it appears that these systems will also be common in private vehicles in a near future, and possibly also mandatory in at least some countries and regions.

The most common approach for vehicle mounted breath testing equipment is to use a mouthpiece to which, after a deep breath, the user should empty his or hers airways. This approach is referred to as active detection. To ensure a correct determination the user should deliver a forced expiration at almost full vital capacity. This requires substantial time and effort, especially for persons with limited capacity. In addition the mouthpiece, or part of the mouthpiece, is often a disposable plastic item for hygienic reasons. This results in cumbersome handling and the use of vast amounts of disposable plastic items, which would be the case if alcolocks become mandatory, is questionable from an environmental viewpoint. An alternative approach is so called passive detection wherein the intoxicating substance is determined from a breath sample taken during the expiration at normal breathing. Disposable mouthpieces are typically not used. However the user may be instructed to breath towards an air inlet or the like. The challenge with passive detection is the low concentration of the substances to be detected and analyzed. An established method is to utilize tracer gases, typically carbon dioxide or water vapor, which are always present in the breath in highly predictable amounts, to both trigger the analysis of the target substance and to facilitate the determination of the target substance concentration value. A further problem, not at least in vehicles, is the uncontrolled environment in the vehicle compartment that includes forced ventilation, the use of AC etc. A still further problem is, if more than the driver is present, to determine if the detected intoxicating substance comes from the driver or from a passenger. WO2017164953 discloses a method and a system for passive breath alcohol concentration measurement using tracer gas detection to facilitate the determination of the target substance concentration value and introduces a method and system to switch from a passive detection to an active detection if required. This also includes instructing a driver to take certain actions. US7736903 discloses a method and a system for passive breath alcohol concentration measurement and discusses the influence of the settings of the HVAC to the ability to perform accurate BrAC measurements

DE 10 2012 202649 A1 discloses a vehicle HVAC system comprising a breath analyzer.

### Summary of the invention

The object of the invention is to provide a breath analyzing system and method of operation that overcomes the drawbacks of prior art methods and systems.

This is achieved by the combined HVAC and breath analyzing system as defined in claim 1, and the method as defined in claim 13.

The vehicle mounted combined HVAC and breath analyzing system according to the invention acts on a compartment of the vehicle and the system comprises:
- a breath analyzer arranged to analyze air from the compartment and to determine a concentration of a target substance in an air sample;
- an HVAC system comprising HVAC system parts to control the air flow in the HVAC system and in the compartment, the HVAC system parts comprising at least one exterior air inlet, at least one interior air inlet and at least one interior air outlet, at least one fan and at least one valve, the HVAC system arranged to have plurality of modes of operation representing different ventilation, heating and cooling operations, and the HVAC system having at least one open position drawing air from the exterior air inlet and at least one closed position drawing air from the interior air inlet, and in both the open and closed position deliver air to a compartment of the vehicle;
- a central control unit arranged to control the breath analyzer and the HVAC system, wherein the central control unit is arranged to override a current mode of the HVAC system to place the HVAC system in a dedicated breath analysis mode, the breath analysis mode comprising controlling the settings of the HVAC system parts to be in positions that improves the possibilities for the breath analyzer to perform the target substance concentration determination, wherein the breath analysis mode comprises at least one time period in which the HVAC system is arranged to be in a closed position, the closed position not allowing any exchange of the air in the compartment with the exterior air and thereby causing a build-up of target substance concentration in the compartment and at least during that time period the central control unit arranges the breath analyzer to analyze air from the compartment. According to the invention the breath analysis mode comprises the HVAC system arranging to rapidly shift to an open position and wherein at least during the transition from the closed to the open position the central control unit arranges the breath analyzer to analyze air from the compartment.

According to one aspect of the invention the combined HVAC and breath analyzing system comprises detections means for detecting the presence of a driver within the compartment and wherein the central control unit is arranged to place the HVAC system in a dedicated breath analysis mode based on the input from the driver presence detection means. The central control unit may be arranged to change valve position and/or activate the breath analyzer based on the input from the driver presence detection means.

According to one aspect of the invention the central control unit is arranged to set or maintain the valve in the first position drawing air from the exterior inlet a first time period after a detection of a driver, setting the valve to the second position drawing air from the interior air inlet a subsequent second time period causing re-circulation of air in the compartment, and returning the valve to the first position after the second time period drawing air from the external inlet and causing purging of air in the compartment; and activating the breath analyzer at least during the second time period. The central control unit may be arranged to activate the breath analyzer at least also during the first time period, wherein the breath analyzer is arranged to measure a baseline.

According to one aspect of the invention the central control unit is arranged to activate the breath analyzer during the first time period, wherein the breath analyzer is arranged to measure a baseline, and the central control unit is arranged to activate the breath analyzer during the second time period, wherein the breath analyzer is arranged to determine the concentration of the target substance in the compartment during the re-circulation, and wherein the breath analyzer is arranged to determine the concentration of the target substance in the compartment during the air purge.

According to one aspect of the invention the breath analyzer is integrated in the HVAC system so that the internal inlet is an inlet to both the HVAC system and the breath analyzer. The breath analyzer may be arranged downstream of the fan and valve of the HVAC system, and according to one aspect the breath analyzer is provided close to an interior air outlet in the vehicle compartment.

According to one aspect of the invention the HVAC system may be placed in a special mode of operation, arranged to draw air backwards from the compartment via the interior air outlet and pass the air to the breath analyzer, thereby facilitating an active detection.

The method of determining a target substance according to the invention using a vehicle mounted combined HVAC and breath analyzing system acting on a compartment of the vehicle, comprises the step of - overriding a current mode of the HVAC system to place the HVAC system in a dedicated breath analysis mode, the breath analysis mode comprising controlling at least one of the HVAC system parts to be in a position that facilitates the breath analyzer to perform a target substance determination, and wherein the breath analysis mode comprises at least one time period in which the HVAC system is in a closed position and thereby causing a build-up of target substance concentration and tracer gas concentration in the compartment and at least during that time period the central control unit arranges the breath analyzer to analyze air from the compartment.

According to one aspect of the invention the method comprises the further steps of:
- Determining if the determination of the target substance is reliable, and if reliable take appropriate action (passing or issuing "alcolock, for example),
- If no reliable determination was possible during the passive detection, initiate an active detection mode,
- Instructing the driver to direct his or hers breath towards the interior air outlet.
- Reverse the air flow of the HVAC system drawing air backwards from the compartment via the interior air outlet, which then acts as a temporary air inlet and passing the air to the breath analyzer.
- Perform a determination of the target substance and return to normal operation of the HVAC.

According to one aspect of the invention the method comprises at least one time period in which the HVAC system is in a closed position and thereby causing a build-up of target substance concentration and tracer gas concentration in the compartment and at least during that time period the central control unit arranges the breath analyzer to analyze air from the compartment.

According to one aspect of the invention the method comprises that the breath analyzer is active and determine the target substance concentration during a shift of the HVAC system from the open position to the closed position or from the closed position to the open position. The dedicated breath analysis mode may comprises changing valve position and/or activate the breath analyzer.

According to one aspect of the invention the method comprises the steps of:
- in a first time period after a detection of a driver the HVAC system is arranged to set or maintain the valve in the first position drawing air from the exterior inlet,
- in a subsequent second time period setting the valve to the second position drawing air from the interior air inlet causing re-circulation of air in the compartment and activating the breath analyzer, and
- returning the valve to the first position after the second time period drawing air from the external inlet and causing purging of air in the compartment.

The breath analyzer may be activated during the first time period, to establish a baseline of the target substance and one or more tracer gases. The breath analyzer may further be active during the air purging step to determine the target substance concentration.

Thanks to the invention the accuracy and the reliability of passive detection in a vehicle is greatly improved. This is of highest importance in getting passive detection to be a realistic alternative to active detection in vehicles.

One advantage of the invention is that disposable mouthpieces are not needed.

One advantage of the invention is that no active exhalation technique is required by the driver.

One further advantage is that the breath analyzing system and the HVAC system may share components like fans, ducts and valves, which lower the cost, complexity and the total size of the combined system compared to having separate and parallel systems. It is also easier to incorporate the breath analyzing system into the interior of the vehicle in an aesthetically pleasing manner. This is of importance in getting breath detection technology to become widely accepted also in private cars.

One further advantage is that the breath analysis system may easily switch from a passive mode to an active or semi-active mode, wherein the driver is instructed to take certain actions, for example direct his or hers breath towards, or actively blowing into a specific vent, acting as an intake of a breath sample.

One further advantage is that the breath analysis system may identify that the driver is about to start the car before the actual starting procedure, for example already then the driver opens the door and prepares for the for the determination by placing the system in a dedicated breath analysis mode beforehand. This shortens the time needed for the determination of the target substance concentration. To keep the analysis time short is of high importance since also a small delay of starting procedure is perceived as very annoying by many drivers.

In the following, the invention will be described in more detail, by way of example only, with regard to non-limiting embodiments thereof, reference being made to the accompanying drawings.

### Brief description of the drawings

Fig. 1a-b are schematic illustrations of the combined HVAC and breath analyzing and system according to the present invention in different modes of operation, a) an open mode and b) a closed mode;
Fig. 2a-d are schematic illustrations of the combined HVAC and breath analyzing system according to different embodiments of the present invention;
Fig. 3 is a graph schematically showing the concentration of breath gases during different modes of operation of the combined HVAC and breath analyzing system; and
Fig. 4 is a flowchart of the method according to the present invention.

### Detailed description

Terms such as "top", "bottom", upper", lower", "below", "above" etc. are used merely with reference to the geometry of the embodiment of the invention shown in the drawings and/or during normal operation of the described device and system and are not intended to limit the invention in any manner.

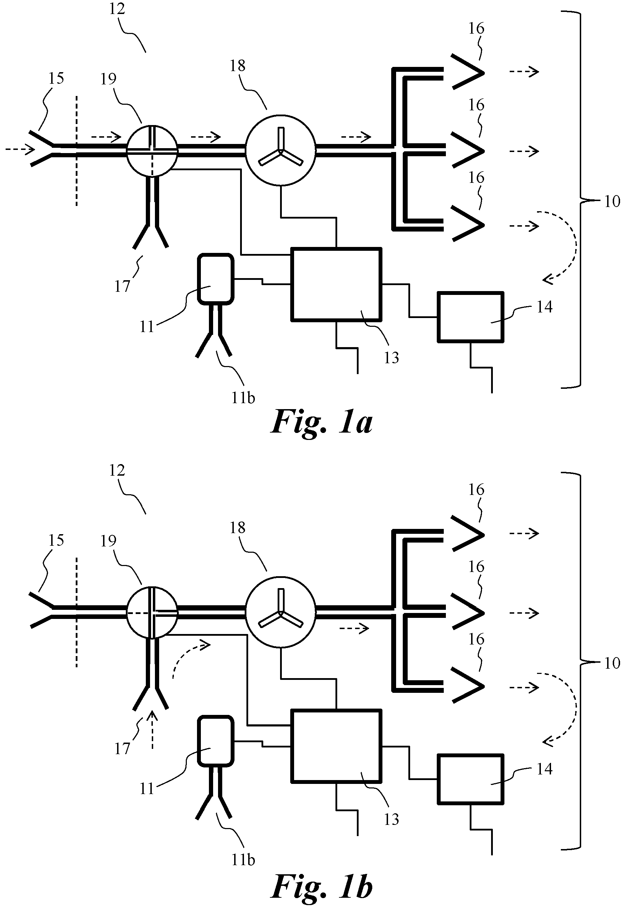

The vehicle mounted combined heating, ventilation, and air conditioning system (HVAC) and breath analyzing system 10 according to the invention is schematically illustrated in Fig. 1 a-b, and comprises a breath analyzer 11 provided with an inlet 11b and an HVAC system 12 of the vehicle. The combined HVAC and breath analyzing system 10 serves the main compartment of the vehicle and may typically, to a large extent, be integrated in the dashboard of the vehicle, although ventilation ducts, a plurality of inlets and outlets etc. extend to other parts of the vehicle. The functionality of a HVAC system is often quite similar between different vehicle models and makers, but the details in the implementation; design etc. may however vary considerably. The breath analyzing system 10 is primarily adapted for passive detection of intoxicating substances or a combination of passive and active detection. Intoxicating substances may typically be alcohol or other illegal or hazardous substances in a driver's breath. The intoxicating substance to be analyzed will be referred to as the target substance.

Passive detection is a term used in the trade to categorize that the breath analysis is performed without the driver blowing into a dedicated mouthpiece or the like. Rather, passive detection aims to perform the breath analysis without the driver taking any specific actions, although some general instructions or conditions, for example requiring the driver to be seated facing forward, close the doors of the vehicle and breathing normally may be needed.

The breath analyzer 11 and HVAC 12 are in connection with a control unit 13. The control unit 13 may typically be a central control unit handling a plurality of functions in the vehicle or a dedicated control unit handling the functionality of the breath analyzer 11 and the HVAC 12. The control unit 13 may further be in connection with a driver detection mechanism 14 which is arranged to detect a driver entering and taking position to drive in the vehicle. The driver detection mechanism 14 may utilize detectors and functions commonly installed in the vehicle such as a detector registration the vehicle door handle being operated, the door opened, the driver seat occupied etc. Further, the control unit 13 may additionally be in connection with, or able to receive information from a plurality of sensors giving indications of the state of the compartment of the vechicle, such as, but not limited to sensors indicating a windows position, existence and number of passengers in the vehicle, inside and outside temperature etc.

The breath analyzer 11 may be a breath analyzer for detection of alcohol in the driver's breath according to US7919754 or US9746454.

The breath analyzer 11 may also be adapted to detect other illegal or hazardous substances. In the following the detection of alcohol will be described as a non-limiting example. Typically, the detection relies on the measurement of the target gas, e.g. alcohol vapor and a tracer gas, e.g. CO₂. The present invention is not limited to a specific breath analyzer. However, it is well-known that passive detection puts high demands on the breath analyzer 11 with regards to sensitivity and speed of detection.

The HVAC system 12 comprises an exterior air inlet 15, a plurality of interior air outlets 16 and an interior air inlet 17, a fan 18 and an arrangement of valves 19 typically maneuverable in a number of different positions or modes giving different type of ventilation of the vehicle compartment. At least a portion of the interior air outlets 16 are typically in the form of vents that can be adjusted with regards to airflow and flow directions. Such vents are typically found in the center console and close to the doors in the compartment of the vehicle. Typical ventilation modes of a HVAC include but is not limited to:
1. Forced ventilation off (fan off) The HVAC system is "open" so that air is entered into exterior air inlet 15 and by no additional force is spread in the compartment via the plurality of interior air outlets 16.
2. Heated forced ventilation (fan on, heater on): The exterior air, actively drawn from the exterior air inlet 15 is heated by a heater or heat exchanger before being distributed into the compartment via the interior air outlets 16.
3. Heated cooled ventilation (fan on, AC on): The exterior air, actively drawn from the exterior air inlet 15 is cooled by an AC before being distributed into the compartment via the interior air outlets 16.
4. Defroster (fan on, heater on): Heated air is directed towards the windscreen and possibly also the side front seat windows.
5. Recirculation (fan on, exterior air inlet closed): With the exterior air inlet 15 closed the compartment air is recirculated within the compartment. This mode is typically used to quickly heat up or cool down the compartment, or to avoid, for a short time period, avoid pollution or smell of the outside air to enter into the compartment.
6. Closed (fan off, exterior air inlet closed): Similar to the recirculation mode, but without forced circulation.

In addition to the basic modes, it is possible, manually or automatically, to direct the air streams in different ways, for example downwards, upwards, towards the face etc.

As is well known the HVAC system may be fully automated so that a user only sets a desired temperature and the system itself choses an appropriate mode based on the desired temperature and inside and outside measured temperature, humidity etc.

The term "valve" should be understood in a functional way as valve means, capable of directing from which inlet the air should be drawn. The skilled person realizes that this may be realized in a number of ways; the most straightforward being a valve in an Y-shaped branched duct. Also having separate fans for the interior air inlet 17 and the exterior air inlet 15 combined with separate valves, for example pressure operated automatic valves, will provide an effective valve functionality that is controllable from the control unit 13. All such variations are apparent for the skilled person and encompassed by the term "valve".

In a similar manner, the term "fan" should be understood in functional manner. The skilled person realizes that a plurality of fans could be used and the implementation in various vehicles could differ significantly.

For the purpose of the present patent application two distinctively different positions is recognized: a HVAC open position wherein external air is drawn from the exterior air inlet 15 to the interior air outlet 16 illustrated in Fig. 1a, and a HVAC closed position wherein air is drawn from the interior air inlet 17 to the interior air outlet 16 illustrated in Fig. 1b, wherein the air flows are indicated with dashed arrows. In both positions the fan may be on or off so that air is forcefully drawn (fan on) from respective exterior air inlet 15 and interior air inlet 17, respectively, or that a mixture occurs without force (fan off).

The combined HVAC and breath analyzing system 10 according to the invention provides a control unit 13 that controls both the HVAC system 12 and the breath analyzer 11 so that the settings of the HVAC system 12 is automatically selected as to speed up and/or increase the accuracy and/or reliability of the detection of alcohol or other specified substances during the time period that the breath analyzer 11 performs the BrAC measurement. This can be described as introducing one or more new modes of the HVAC system 12, referred to as dedicated breath analysis modes, to optimize the breath analysis performed by the breath analyzer 11 and that the control unit 13 is arranged to override a current mode of the HVAC system to place the HVAC system in a dedicated breath analysis mode that facilitate the determination of the target substance concentration.

According to one embodiment of the invention, the breath analysis mode comprises at least one time period in which the HVAC system 12 is in a closed position and at least during that time period the control unit 13 arranges the breath analyzer 11 to analyze air from the compartment. After the breath analysis mode, the HVAC system may return to the mode it had before the breath analysis mode. Preferably the control unit 13 initiates the breath analysis mode a time period before the breath analyzer 11 actually performs the BrAC measurement. This is to facilitate build up of concentrations of the gases to be analyzed and/or to let a stable situation of the airflow in the compartment be established before the measurement is done. According to one embodiment of the invention the breath analysis mode of the HVAC system 12 comprises the HVAC system 12 being in a HVAC closed position not allowing exterior air to enter into the compartment during the breath analyzer 11 performing the BrAC measurement. The HVAC closed position may incorporate the fan of the HVAC system being on for circulating the air of the compartment. The time period, which represents the build-up phase, may be a predetermined time period. The length of the time period may be determined taken into account physical considerations - in order for a build up to be meaningful the person should be able to take a few breaths. Typically and preferably the time period for the build-up phase is in the order of 10s to 1 min. Alternatively, according to another embodiment the time period of the build-up phase is dynamic and determined from measurements done by the breath analyzer 11. The measurements may be continuous, periodic or pointwise during the build-up phase and may include, but is not limited to:
- measuring the tracer gas concentration and end the build-up phase when the tracer gas concentration exceeds a predetermined level, and/or
- analyzing the statistics of target gas measurements and end the build-up when the confidence intervals are within a predetermined range.

According to one embodiment of the invention the breath analysis mode of the HVAC system comprises initiating a change in the air composition of the compartment during a BrAc measurement period. This is achieved by that the control unit 13 arranges to change the arrangement of valves 19 to one of the first and second positions and thereby initiate a change of the air composition of the compartment, and in relation to the change in valve position activate the breath analyzer to analyze air from the compartment. Due to differences in response to such a change of alcohol and the tracer gas information may be extracted from measurement data giving an improved detection of alcohol.

According to one embodiment the HVAC system is arranged to be in a closed position and thereby causing a build-up of target substance concentration and tracer gas concentration in the compartment. Subsequently the HVAC system is arranged to rapidly shift to an open position and wherein at least during the transition from the closed to the open position the control unit 13 arranges the breath analyzer to analyze air from the compartment.

According to one embodiment the combined HVAC and breath analyzing system according to comprises detections means for detecting the presence of a driver within the compartment and wherein the control unit 13 arranges to change valve position and/or activate the breath analyzer based on the input from the driver presence detection means. Also other detectors and indications could be taken into account, for example sensors or switches determining that doors are closed, windows closed etc. If the compartment determined as a closed compartment the driver might be instructed to close doors and windows, for example, before the measurement process is initiated.

According to one embodiment the combined HVAC and breath analyzing system 10 the control unit 13 arranges to set, or maintain, the HVAC system 12 in the open position drawing air from the exterior inlet a first time period after a person has occupied the driver seat of the vehicle, setting the HVAC system 12 in the closed position drawing air from the interior air inlet a subsequent second time period causing re-circulation of air in the compartment, and returning to the HVAC open position after the second time period drawing air from the external inlet and causing purging of air in the compartment; and activating the breath analyzer at least during the second time period. During the first time period the breath analyzer may be arranged to measure a baseline. The intake of fresh air via the exterior inlet ensures that the baseline values for alcohol and tracer gas are relatively stable and predictable values. The first time period is typically 5-30s and the second time period is in the order of 1-3 minutes.

According to one embodiment, after the first time period the control unit 13 controls the HVAC system 12 to shift to a closed mode, i.e. change position of the valve 19 so that air is drawn from interior inlet 17, thereby creating a recirculation of air in the vehicle compartment. This represents a concentration build-up phase of the measurement cycle. During the build-up phase the concentration of the tracer gas and the target substance, if at all present in the breath of the driver and/or passenger(s), will increase continuously due to the repeated and periodic exhalations from the person(s) in the vehicle compartment. A reasonable assumption is that the increase in concentrations is linear for both the target substance and the tracer gas. Since the concentration, especially for the target substance, may be very low the increase in concentration may not be observable for a first time interval during the build-up phase, due to the concentration being below a detection limit. For a second time interval only, a continuous increase is observable, referred to as the continuously increasing target substance concentration interval. The determination of a continuously increasing target substance concentration may be used as an indication of a reliable determination.

According to one embodiment the first time period is a predetermined time period. Alternatively the length of first time period is determined based on the baseline measurement, in that the initial phase may end upon an indication from the breath analyzer that a stable measurement of the baseline has been acquired.

Due to the recirculation of the air in the vehicle the concentration of exhaled gases increases in the vehicle compartment; both target gas and tracer gas will build up, facilitating the detection of the target substance. The breath analyzer 11 measures the target substance and the tracer gas during the concentration build up phase. The measurement may be continuous or pointwise during the phase. After a second time period the control unit 13 controls the HVAC system 12 to switch the valve 19 from the second position to the first position, i.e. open to the exterior air inlet 15 so that fresh air will enter the compartment.

According to one embodiment the second time period is a predetermined time period. Alternatively the length of second time period is determined based on the measurement of the tracer gas, the target substance or the tracer gas and target substance. The ending of the second time period (ending of the build-up phase) may be based on that a continuously increasing target substance concentration has been determined, indicative of that the build-up concentration is above a detection level and that the concentration determination is a reliable determination. According to one embodiment the target substance concentration that is presented to the driver, or used for the "alcolock" function, for example, is based at least partly on the continuously increasing target substance concentration interval.

Typically the breath analyzer 11 is controlled to make measurement also during and immediately after, the shift from re-circulation to open ventilation as the difference in decline of target gas and tracer gas concentrations may be used in establishing the target gas concentration in the driver's breath.

According to one embodiment of the invention the breath analyzer 11 is an integral part of the HVAC system 12 so that the breath analyzer 11 is directly exposed to the airflow created in the HVAC system 12.

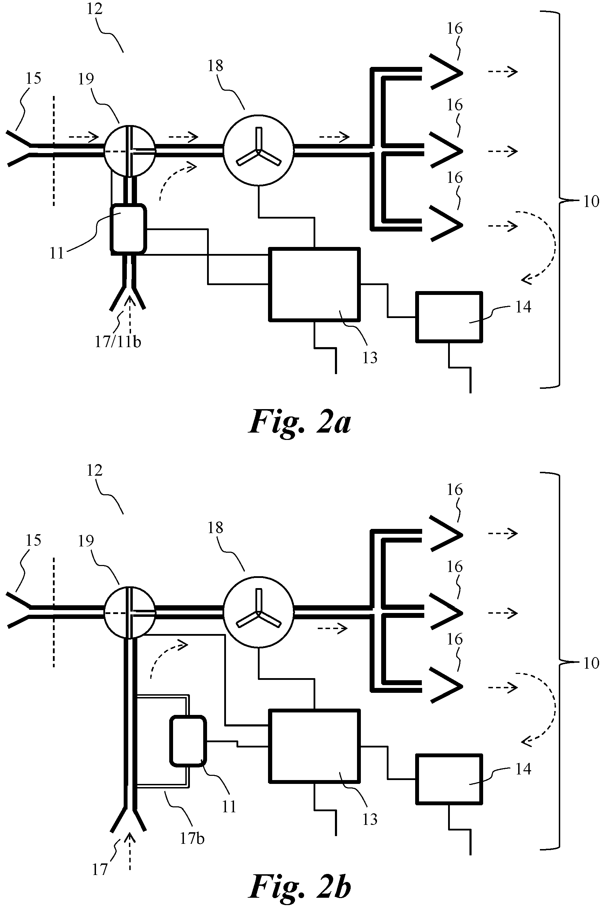

According to one embodiment, schematically illustrated in Fig. 2a, the breath analyzer 11 is arranged in, or in close connection to the interior air inlet 17, so that the fan 18 of the HVAC system 12, draws air through the breath analyzer 11 then the valve 19 is in its second position, i.e. during re-circulation. Alternatively, as schematically illustrated in Fig. 2b, the breath analyzer 11 is provided in a side interior air channel 17b arranged in parallel to the interior air inlet 17 which provides a major air flow and the side interior air channel 17b providing a minor air flow to the breath analyzer 11. Alternatively the breath analyzer 11 have its own air inlet 11b, but in fluid connection with the interior air inlet so that the air is drawn also into the breath analyzer 11 by the HVAC fan 18.

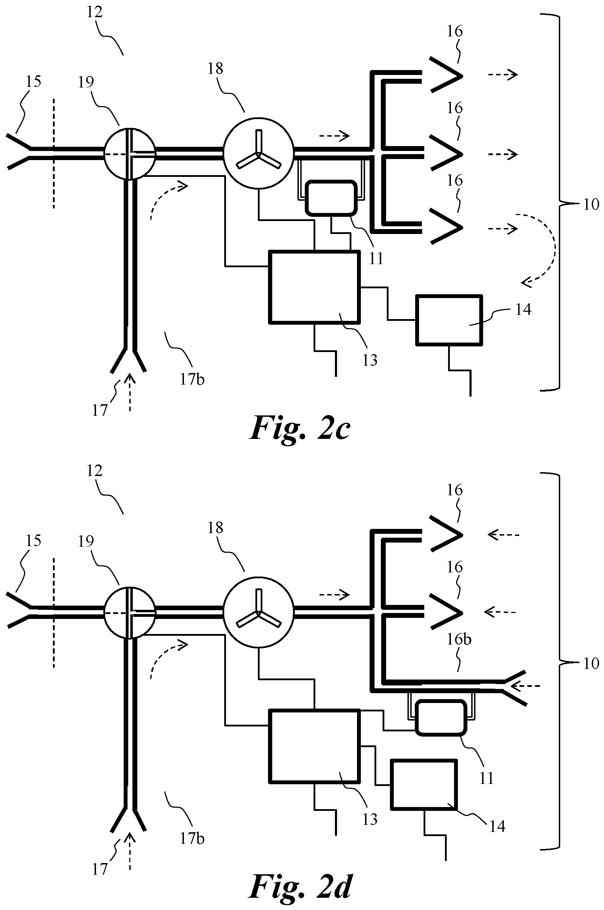

According to one embodiment of the invention schematically illustrated in Fig. 2c, the breath analyzer 11 is provided in the HVAC system 12 downstream of the fan 18. This ensures that the breath analyzer 11 will receive a controllable flow of air both during the initial phase and the concentration build up phase.

According to one embodiment of the invention schematically illustrated in Fig. 2d, the breath analyzer 11 is provided in the HVAC system 12 after the fan 18 and close to one of the interior air outlet 16. This enables that the fan 18 may be driven backwards to create a backwards flow of air into the HVA system. In that the interior air outlet 16b temporarily acts as an air inlet 16b.

According to one embodiment of the invention if the breath analysis during a dedicated breath analysis mode, which is a passive detection. indicates either that the that the breath analysis could not be performed correctly the driver is instructed to direct his or hers breath towards a specific vent of the HVAC system 12 or towards a dedicated breath analysis inlet. Indications of uncorrected determination may include, but is not limited to: very low concentrations of tracer gas- indicating a possible tampering attempt, very high target substance concentration indicating more persons present in the vehicle compartment being intoxicated , or a fluctuating target substance concentration, for example, The combined breath analyzing and HVAC system 12 then enters into an active breath analysis mode actively drawing air comprising the breath sample into the specific vent or dedicated breath analysis inlet and passes it to the breath analyzer within the HVAC system 12. Alternatively the driver is instructed to actively blow into the specific vent/inlet. A dedicated replace mouthpiece, connectable to the dedicated inlet, could be utilized, but is according to one embodiment not required.

Referring to the ventilation modes of a HVAC listed above the combined breath analyzis and HVAC system 12 and method have one or more of the following operational modes, referred to as dedicated breath analysis modes:
7. Concentration build-up passive breath analysis mode (fan on, exterior air inlet closed (recirculation), breath analyzer active): With the exterior air inlet 15 closed the compartment air is recirculated within the compartment resulting in the described build-up of breath gases.
8. Active breath analysis mode (fan on, exterior air inlet closed (recirculation), breath analyzer active): The HVAC system 12 is arranged to draw air into a specific inlet and the driver is instructed to face the inlet or actively blow into the inlet, and pass the air to the breath analyzer within the HVAC system 12.
9. A baseline detection mode (fan on, exterior inlet open, outlet to compartment closed, breath analyzer active). The HVAC system 12 is arranged to draw air outside air through its ducts and to the breath analyzer in order to provide a stable and reproducible baseline measurement.
10. A purging mode (fan on, exterior inlet open, outlet to compartment open, breath analyzer active or inactive). The HVAC system 12 is arranged to actively purge the compartment in order to facilitate upcoming measurements. The breath analyzer may be active to, for example to indicate then the level of CO₂ is at a predetermined low value indicative of a sufficiently vented compartment.

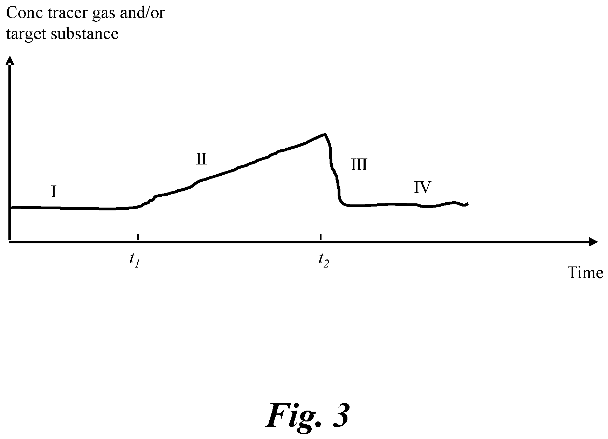

Illustrated in Fig. 3 is a sequence of dedicated breath analysis modes according to one embodiment starting with a purging mode I initialized upon the detection of a driver entering the vehicle and occupying the driver seat. The combined HVAC and breath analyzing system turns to the concentration build-up passive breath analysis mode, after the first time period *t₁*, resulting in the build-up of tracer gas and target substances concentration build-up as discussed above. A rapid change back to the purge mode, after the second time period *t₂*, the concentration of the target substance and tracer gas go back to their baseline values IV. To keep the breath analyzer active for the complete sequence may provide information that is useful in the determination of the target substance concentration and/or to identify an unknown target substance.

The method of determining a target substance according to the invention uses the vehicle mounted combined HVAC and breath analyzing system described above. According to the method a current mode of the HVAC system 12 is overridden to place the HVAC system 12 in a dedicated breath analysis mode, the breath analysis mode comprising controlling at least one of the HVAC system 12 parts to be in a position that facilitates the breath analyzer to perform a target substance determination.

Embodiments of the method correspond to the embodiments of the combined HVAC and breath analyzing system and the dedicated breath analysis modes described above.

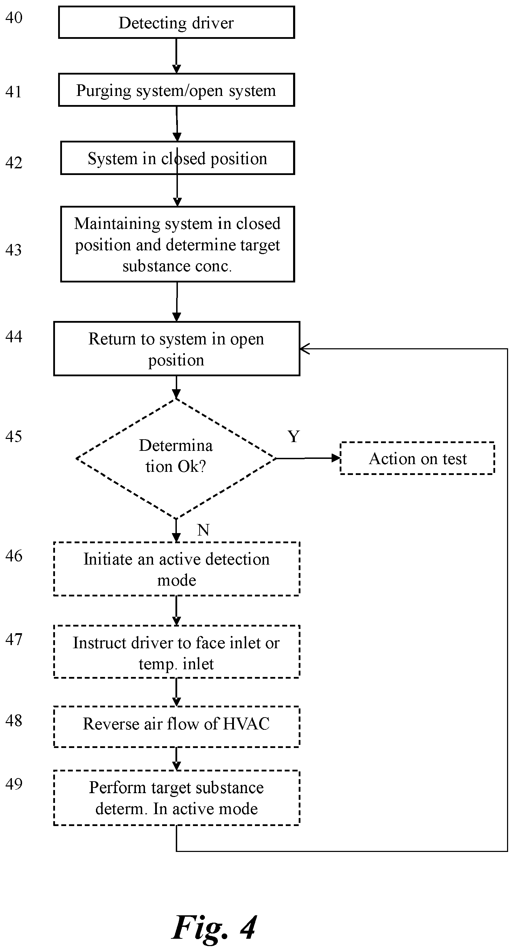

One embodiment of the method according to the invention, described with reference to the flowchart of Fig 4, comprises the steps of:
40: detecting the presence of a driver within the compartment or that the driver is about to enter the vehicle,
41: purging the vehicle compartment for a first time period by placing the HVAC system 12 in an open position,
42: placing the HVAC system 12 in a closed position thereby the compartment air is recirculated within the compartment resulting in the described build-up of breath gases,
43: maintaining the HVAC system 12 in a closed position for a second time period wherein the breath analyzer is active,
44: placing the HVAC system 12 in an open position.

According to one embodiment the method comprises the further steps of:
45: Determining if the determination of the target substance is reliable, and if reliable take appropriate action (passing or issuing "alcolock, for example),
46: If no reliable determination was possible during the passive detection, initiate an active detection mode,
47: Instructing the driver to direct his or hers breath towards the interior air outlet.
48: Reverse the air flow of the HVAC system 12 drawing air backwards from the compartment via the interior air outlet, which then acts as a temporary air inlet and passing the air to the breath analyzer.
49: Perform a determination of the target substance and return to normal operation of the HVAC.

The method and system have above been described primarily in a scenario wherein the driver enters and is about to start the vehicle. The system and method can equally well be used during operation/driving with the omission of certain steps, which should be obvious to the skilled person, for example detecting the presence of the driver. Such test during operation/driving may be of importance to control that the driver is not using alcohol while driving after the initial test (and possible passing the initial test), for example.

The embodiments described above are to be understood as illustrative examples of the system and method of the present invention. It will be understood that those skilled in the art that various modifications, combinations and changes may be made to the embodiments, within the scope of the appended claims.

## Claims

1. A vehicle mounted combined HVAC and breath analyzing system (10) acting on a compartment of the vehicle, the combined HVAC and breath analyzing system (10) comprising:
- a breath analyzer (11) arranged to analyze air from the compartment and to determine a concentration of a target substance in an air sample ;
- an HVAC system (12) comprising HVAC system parts to control the air flow in the HVAC system (12) and in the compartment, the HVAC system (12) parts comprising at least one exterior air inlet (15), at least one interior air inlet (17) and at least one interior air outlet (16), at least one fan (18) and at least one valve (19), the HVAC system (12) arranged to have plurality of modes of operation representing different ventilation, heating and cooling operations achievable by different settings or sequences of settings of the HVAC system parts, and the HVAC system (12) having at least one open position drawing air from the exterior air inlet (15) and at least one closed position drawing air from the interior air inlet (17), and in both the open and closed position deliver air to a compartment of the vehicle; comprising
- a central control unit (13) arranged to control both the breath analyzer (11) and the HVAC system;
wherein the central control unit (13) is arranged to override a current mode of the HVAC system (12) to place the HVAC system (12) in a dedicated breath analysis mode, the breath analysis mode comprising controlling the settings of the HVAC system (12) parts to be in positions that improves the possibilities for the breath analyzer (11) to perform the target substance concentration determination, wherein the breath analysis mode comprises at least one time period in which the HVAC system (12) is arranged to be in a closed position, the closed position not allowing any exchange of the air in the compartment with the exterior air and thereby causing a build-up of target substance concentration in the compartment and at least during that time period the central control unit (13) arranges the breath analyzer (11) to analyze air from the compartment, **characterized in that** the HVAC system (12) is arranged to rapidly shift to an open position and wherein at least during the transition from the closed to the open position the central control unit (13) arranges the breath analyzer (11) to analyze air from the compartment.

2. The vehicle mounted combined HVAC and breath analyzing system (10) according to claim 1, wherein the rapid shift is arranged to be performed after the time period in the closed position.

3. The vehicle mounted combined HVAC and breath analyzing system (10) according to any of the preceding claims, wherein the central control unit (13) is arranged to set or maintain HVAC system (12) in a first position drawing air from the exterior inlet a first time period, setting the HVAC system (12) to the closed position a subsequent second time period, and returning the HVAC system (12) to the first position after the second time period drawing air from the external inlet and causing purging of air in the compartment; and activating the breath analyzer (11) at least during the second time period.

4. The vehicle mounted combined HVAC and breath analyzing system (10) according to claim 3, wherein the central control unit (13) is arranged to activate the breath analyzer (11) at least also during the first time period, wherein the breath analyzer (11) is arranged to measure a baseline.

5. The vehicle mounted combined HVAC and breath analyzing system (10) according to any of the preceding claims, further comprising detections means for detecting the presence of a driver within the compartment and wherein the central control unit (13) is arranged to place the HVAC system (12) in a dedicated breath analysis mode based on the input from the driver presence detection means.

6. The vehicle mounted combined HVAC and breath analyzing system (10) according to claim 5, wherein the central control unit (13) is arranged to change valve position and/or activate the breath analyzer (11) based on the input from the driver presence detection means.

7. The vehicle mounted combined HVAC and breath analyzing system (10) according to claim 3 and 6, wherein the central control unit (13) is arranged to set or maintain the valve in the first position drawing air from the exterior inlet a first time period after a detection of a driver.

8. The vehicle mounted combined HVAC and breath analyzing system (10) according to claim 4, wherein the central control unit (13) is arranged to activate the breath analyzer (11) during the first time period, wherein the breath analyzer (11) is arranged to measure a baseline, and the central control unit (13) is arranged to activate the breath analyzer (11) during the second time period, wherein the breath analyzer (11) is arranged to determine the concentration of the target substance in the compartment during the re-circulation, and wherein the breath analyzer (11) is arranged to determine the concentration of the target substance in the compartment during the air purge.

9. The vehicle mounted combined HVAC and breath analyzing system (10) according to any of the preceding claims, wherein the breath analyzer (11) is integrated in the HVAC system (12) so that the internal inlet is an inlet to both the HVAC system (12) and the breath analyzer.

10. The vehicle mounted combined HVAC and breath analyzing system (10) according to claim 9, wherein the breath analyzer (11) is arranged downstream of the fan (18) and valve of the HVAC system.

11. The vehicle mounted combined HVAC and breath analyzing system (10) according to any of the preceding claims, wherein the HVAC system (12) is, in a special mode of operation, arranged to draw air backwards from the compartment via the interior air outlet (16) and pass the air to the breath analyzer.

12. The vehicle mounted combined HVAC and breath analyzing system (10) according to any of the preceding claims, wherein the breath analyzer (11) is arranged to measure a target substance concentration and a tracer gas concentration.

13. A method of determining a target substance using a vehicle mounted combined HVAC and breath analyzing system (10) acting on a compartment of the vehicle, wherein the combined HVAC and breath analyzing system (10) comprises a breath analyzer (11) arranged to analyze air from the compartment and to determine a target substance and a tracer gas in an air sample, an HVAC system (12) comprising HVAC system parts to control the air flow in the HVAC system (12) and in the compartment, the HVAC system parts comprising at least one exterior air inlet (15), at least one interior air inlet (17) and at least one interior air outlet (16), at least one fan (18) and at least one valve, the HVAC system (12) arranged to have plurality of modes of operation representing different ventilation, heating and cooling operations, and a central control unit (13) arranged to control with the breath analyzer (11) and the HVAC system, the method comprising overriding a current mode of the HVAC system (12) to place the HVAC system (12) in a dedicated breath analysis mode, the breath analysis mode comprising controlling at least one of the HVAC system parts to be in a position that facilitates the breath analyzer (11) to perform a target substance determination, and wherein the breath analysis mode comprises at least one time period in which the HVAC system (12) is in a closed position and thereby causing a build-up of target substance concentration and tracer gas concentration in the compartment and at least during that time period the central control unit (13) arranges the breath analyzer (11) to analyze air from the compartment, **characterized in that** the breath analyzer (11) is active and determine the target substance concentration during a shift of the HVAC system (12) from the open position to the closed position or from the closed position to the open position.

14. The method according to claim 13, further comprising the steps of
- detecting the presence of a driver; and
- placing the HVAC system (12) in a dedicated breath analysis mode based on the detection of a driver.

15. The method according to claim 13, wherein placing the HVAC system (12) in a dedicated breath analysis mode comprises changing valve position and/or activate the breath analyzer.

16. The method according to claim 13, comprising the steps of
- in a first time period the HVAC system (12) is arranged to be set or to be maintained in a first position drawing air from the exterior inlet,
- in a subsequent second time period setting the HVAC system (12) to the closed position drawing air from the interior air inlet (17) causing re-circulation of air in the compartment and activating the breath analyzer, and
- returning the HVAC system (12) to the first position after the second time period drawing air from the external inlet and causing purging of air in the compartment.

17. The method according to claim 16, wherein the breath analyzer (11) is activated during the first time period, wherein the breath analyzer (11) measures a baseline.

18. The method according to claim 17, further comprising the breath analyzer (11) measuring during the air purging step.

19. The method according to any of claims 13 to 18, further comprising the HVAC system (12) drawing air backwards from the compartment via the interior air outlet (16) and passing the air to the breath analyzer, and instructing the driver to direct his or hers breath towards the interior air outlet (16).

20. The method according to any of claims14 to 19, further comprising the HVAC system (12) drawing air backwards from the compartment via a dedicated inlet and passing the air to the breath analyzer, and instructing the driver to direct his or hers breath towards the dedicated inlet.

## Patentansprüche

1. Fahrzeugmontiertes kombiniertes HLK- und Atemanalysesystem (10), das auf einen Fahrgastraum des Fahrzeugs wirkt, wobei das kombinierte HLK- und Atemanalysesystem (10) Folgendes umfasst:
- einen Atemanalysator (11), der eingerichtet ist, um Luft aus dem Fahrgastraum zu analysieren und eine Konzentration einer Zielsubstanz in einer Luftprobe zu bestimmen;
- ein HLK-System (12), das HLK-Systemteile umfasst, um den Luftstrom in dem HLK-System (12) und in dem Fahrgastraum zu steuern, wobei die Teile des HLK-Systems (12) mindestens einen Außenlufteinlass (15), mindestens einen Innenlufteinlass (17) und mindestens einen Innenluftauslass (16), mindestens einen Lüfter (18) und mindestens ein Ventil (19) umfassen, wobei das HLK-System (12) eingerichtet ist, eine Vielzahl von Betriebsmodi aufzuweisen, die unterschiedliche Lüftungs-, Heiz- und Kühlvorgänge darstellen, die durch unterschiedliche Einstellungen oder Einstellungssequenzen der HLK-Systemteile erzielbar sind, und wobei das HLK-System (12) mindestens eine offene Position, die Luft aus dem Außenlufteinlass (15) ansaugt, und mindestens eine geschlossene Position, die Luft aus dem Innenlufteinlass (17) ansaugt, aufweist und sowohl in der offenen als auch in der geschlossenen Position Luft zu einem Fahrgastraum des Fahrzeugs abgibt;
umfassend
- eine zentrale Steuereinheit (13), die eingerichtet ist, sowohl den Atemanalysator (11) als auch das HLK-System zu steuern;
wobei die zentrale Steuereinheit (13) eingerichtet ist, einen aktuellen Modus des HLK-Systems (12) zu übersteuern, um das HLK-System (12) in einen dedizierten Atemanalysemodus zu versetzen, wobei der Atemanalysemodus das Steuern der Einstellungen der Teile des HLK-Systems (12) derart umfasst, dass sie sich in Positionen befinden, die die Möglichkeiten für den Atemanalysator (11) verbessern, die Bestimmung der Zielsubstanzkonzentration durchzuführen, wobei der Atemanalysemodus mindestens einen Zeitraum umfasst, in dem das HLK-System (12) so eingerichtet ist, dass es sich in einer geschlossenen Position befindet, wobei die geschlossene Position keinen Austausch der Luft in dem Fahrgastraum mit der Außenluft zulässt und dadurch einen Aufbau der Zielsubstanzkonzentration in dem Fahrgastraum verursacht und mindestens während dieses Zeitraums die zentrale Steuereinheit (13) den Atemanalysator (11) veranlasst, Luft aus dem Fahrgastraum zu analysieren, **dadurch gekennzeichnet, dass**
das HLK-System (12) dazu eingerichtet ist, schnell in eine offene Position zu wechseln, und wobei mindestens während des Übergangs von der geschlossenen in die offene Position die zentrale Steuereinheit (13) den Atemanalysator (11) veranlasst, Luft aus dem Fahrgastraum zu analysieren.

2. Fahrzeugmontiertes kombiniertes HLK- und Atemanalysesystem (10) nach Anspruch 1, wobei der schnelle Wechsel so eingerichtet ist, dass er nach dem Zeitraum in der geschlossenen Position durchgeführt wird.

3. Fahrzeugmontiertes kombiniertes HLK- und Atemanalysesystem (10) nach einem der vorhergehenden Ansprüche, wobei die zentrale Steuereinheit (13) dazu eingerichtet ist, das HLK-System (12) in eine erste Position, in der Luft aus dem Außeneinlass in einem ersten Zeitraum angesaugt wird, einzustellen oder in dieser zu halten, das HLK-System (12) in einem nachfolgenden zweiten Zeitraum in die geschlossene Position einzustellen und das HLK-System (12) nach dem zweiten Zeitraum in die erste Position zurückzuführen, um Luft aus dem Außeneinlass anzusaugen und ein Spülen von Luft in dem Fahrgastraum zu bewirken; und den Atemanalysator (11) mindestens während des zweiten Zeitraums zu aktivieren.

4. Fahrzeugmontiertes kombiniertes HLK- und Atemanalysesystem (10) nach Anspruch 3, wobei die zentrale Steuereinheit (13) dazu eingerichtet ist, den Atemanalysator (11) mindestens auch während des ersten Zeitraums zu aktivieren, wobei der Atemanalysator (11) dazu eingerichtet ist, einen Ausgangswert zu messen.

5. Fahrzeugmontiertes kombiniertes HLK- und Atemanalysesystem (10) nach einem der vorhergehenden Ansprüche, ferner umfassend Erkennungsmittel zum Erkennen der Anwesenheit eines Fahrers in dem Fahrgastraum und wobei die zentrale Steuereinheit (13) dazu eingerichtet ist, das HLK-System (12) basierend auf der Eingabe von dem Fahreranwesenheitserkennungsmittel in einen dedizierten Atemanalysemodus zu versetzen.

6. Fahrzeugmontiertes kombiniertes HLK- und Atemanalysesystem (10) nach Anspruch 5, wobei die zentrale Steuereinheit (13) dazu eingerichtet ist, die Ventilposition zu ändern und/oder den Atemanalysator (11) basierend auf der Eingabe von dem Fahreranwesenheitserkennungsmittel zu aktivieren.

7. Fahrzeugmontiertes kombiniertes HLK- und Atemanalysesystem (10) nach Anspruch 3 und 6, wobei die zentrale Steuereinheit (13) dazu eingerichtet ist, das Ventil in die erste Position, in der Luft aus dem Außeneinlass in einem ersten Zeitraum nach einer Erkennung eines Fahrers angesaugt wird, einzustellen oder in dieser zu halten.

8. Fahrzeugmontiertes kombiniertes HLK- und Atemanalysesystem (10) nach Anspruch 4, wobei die zentrale Steuereinheit (13) dazu eingerichtet ist, den Atemanalysator (11) während des ersten Zeitraums zu aktivieren, wobei der Atemanalysator (11) dazu eingerichtet ist, einen Ausgangswert zu messen, und die zentrale Steuereinheit (13) dazu eingerichtet ist, den Atemanalysator (11) während des zweiten Zeitraums zu aktivieren, wobei der Atemanalysator (11) dazu eingerichtet ist, die Konzentration der Zielsubstanz in dem Fahrgastraum während der Rezirkulation zu bestimmen, und wobei der Atemanalysator (11) dazu eingerichtet ist, die Konzentration der Zielsubstanz in dem Fahrgastraum während der Luftspülung zu bestimmen.

9. Fahrzeugmontiertes kombiniertes HLK- und Atemanalysesystem (10) nach einem der vorhergehenden Ansprüche, wobei der Atemanalysator (11) in das HLK-System (12) integriert ist, sodass der innere Einlass ein Einlass sowohl für das HLK-System (12) als auch für den Atemanalysator ist.

10. Fahrzeugmontiertes kombiniertes HLK- und Atemanalysesystem (10) nach Anspruch 9, wobei der Atemanalysator (11) stromabwärts des Lüfters (18) und des Ventils des HLK-Systems eingerichtet ist.

11. Fahrzeugmontiertes kombiniertes HLK- und Atemanalysesystem (10) nach einem der vorhergehenden Ansprüche, wobei das HLK-System (12) in einem speziellen Betriebsmodus eingerichtet ist, Luft nach hinten aus dem Fahrgastraum über den Innenluftauslass (16) anzusaugen und die Luft zu dem Atemanalysator zu leiten.

12. Fahrzeugmontiertes kombiniertes HLK- und Atemanalysesystem (10) nach einem der vorhergehenden Ansprüche, wobei der Atemanalysator (11) eingerichtet ist, eine Zielsubstanzkonzentration und eine Prüfgaskonzentration zu messen.

13. Verfahren zum Bestimmen einer Zielsubstanz unter Verwendung eines fahrzeugmontierten kombinierten HLK- und Atemanalysesystems (10), das auf einen Fahrgastraum des Fahrzeugs wirkt, wobei das kombinierte HLK- und Atemanalysesystem (10) einen Atemanalysator (11) umfasst, der eingerichtet ist, Luft aus dem Fahrgastraum zu analysieren und eine Zielsubstanz und ein Prüfgas in einer Luftprobe zu bestimmen, wobei ein HLK-System (12) HLK-Systemteile zum Steuern des Luftstroms in dem HLK-System (12) und in dem Fahrgastraum umfasst, wobei die HLK-Systemteile mindestens einen Außenlufteinlass (15), mindestens einen Innenlufteinlass (17) und mindestens einen Innenluftauslass (16), mindestens einen Lüfter (18) und mindestens ein Ventil umfassen, wobei das HLK-System (12) eingerichtet ist, eine Vielzahl von Betriebsmodi, die verschiedene Lüftungs-, Heiz- und Kühlvorgänge darstellen, und eine zentrale Steuereinheit (13), die eingerichtet ist, den Atemanalysator (11) und das HLK-System zu steuern, aufzuweisen, wobei das Verfahren das Übersteuern eines aktuellen Modus des HLK-Systems (12) umfasst, um das HLK-System (12) in einen dedizierten Atemanalysemodus zu versetzen, wobei der Atemanalysemodus das Steuern mindestens eines der HLK-Systemteile derart umfasst, dass es sich in einer Position befindet, die es dem Atemanalysator (11) ermöglicht, eine Zielsubstanzbestimmung durchzuführen, und wobei der Atemanalysemodus mindestens einen Zeitraum umfasst, in dem sich das HLK-System (12) in einer geschlossenen Position befindet und dadurch einen Aufbau der Zielsubstanzkonzentration und Prüfgaskonzentration in dem Fahrgastraum bewirkt, und mindestens während dieses Zeitraums die zentrale Steuereinheit (13) den Atemanalysator (11) veranlasst, Luft aus dem Fahrgastraum zu analysieren, **dadurch gekennzeichnet, dass** der Atemanalysator (11) aktiv ist und die Zielsubstanzkonzentration während eines Wechsels des HLK-Systems (12) von der offenen Stellung in die geschlossene Stellung oder von der geschlossenen Stellung in die offene Stellung bestimmt.

14. Verfahren nach Anspruch 13, ferner die folgenden Schritte umfassend:
- Erkennen der Anwesenheit eines Fahrers; und
- Versetzen des HLK-Systems (12) in einen dedizierten Atemanalysemodus basierend auf der Erkennung eines Fahrers.

15. Verfahren nach Anspruch 13, wobei das Versetzen des HLK-Systems (12) in einen dedizierten Atemanalysemodus das Ändern der Ventilposition und/oder das Aktivieren des Atemanalysators umfasst.

16. Verfahren nach Anspruch 13, die folgenden Schritte umfassend:
- in einem ersten Zeitraum ist das HLK-System (12) dazu eingerichtet, in eine erste Position, in der Luft aus dem Außeneinlass angesaugt wird, eingestellt zu werden oder in dieser gehalten zu werden,
- in einem nachfolgenden zweiten Zeitraum Einstellen des HLK-Systems (12) in die geschlossene Position, in der Luft aus dem Innenlufteinlass (17) angesaugt wird, wodurch eine Luftzirkulation in dem Fahrgastraum bewirkt wird und der Atemanalysator aktiviert wird, und
- Zurückführen des HLK-Systems (12) nach dem zweiten Zeitraum in die erste Position, in der Luft aus dem externen Einlass angesaugt und die Luft in dem Fahrgastraum gespült wird.

17. Verfahren nach Anspruch 16, wobei der Atemanalysator (11) während des ersten Zeitraums aktiviert wird, wobei der Atemanalysator (11) einen Ausgangswert misst.

18. Verfahren nach Anspruch 17, ferner umfassend den Atemanalysator (11), der während des Schrittes des Luftspülens misst.

19. Verfahren nach einem der Ansprüche 13-18, ferner umfassend das HLK-System (12), das Luft nach hinten aus dem Fahrgastraum über den Innenluftauslass (16) ansaugt und die Luft zum Atemanalysator leitet und den Fahrer/die Fahrerin anweist, seinen oder ihren Atem auf den Innenluftauslass (16) zu richten.

20. Verfahren nach einem der Ansprüche 14 bis 19, ferner umfassend das HLK-System (12), das Luft nach hinten aus dem Fahrgastraum über einen dedizierten Einlass ansaugt und die Luft zum Atemanalysator leitet und den Fahrer/die Fahrerin anweist, seinen oder ihren Atem auf den dedizierten Einlass zu richten.

## Revendications

1. Système d'analyse d'haleine et de CVC combinés montés sur véhicule (10) agissant sur un compartiment du véhicule, le système d'analyse d'haleine et de CVC combinés (10) comprenant :
- un analyseur d'haleine (11) agencé pour analyser l'air provenant du compartiment et pour déterminer une concentration d'une substance cible dans un échantillon d'air ;
- un système CVC (12) comprenant des parties du système CVC pour commander le flux d'air dans le système CVC (12) et dans le compartiment, les parties du système CVC (12) comprenant au moins une entrée d'air extérieur (15), au moins une entrée d'air intérieur (17) et au moins une sortie d'air intérieur (16), au moins un ventilateur (18) et au moins une vanne (19), le système CVC (12) étant agencé pour avoir une pluralité de modes de fonctionnement représentant différentes opérations de ventilation, de chauffage et de refroidissement réalisables par différents réglages ou séquences de réglages des parties du système CVC, et le système CVC (12) ayant au moins une position ouverte aspirant l'air de l'entrée d'air extérieur (15) et au moins une position fermée aspirant l'air de l'entrée d'air intérieur (17) et, dans les positions ouverte et fermée, fournit de l'air à un compartiment du véhicule ;
comprenant
- une unité de commande centrale (13) agencée pour commander à la fois l'analyseur d'haleine (11) et le système CVC ;
dans lequel l'unité de commande centrale (13) est agencée pour remplacer un mode actuel du système CVC (12) pour placer le système CVC (12) dans un mode d'analyse d'haleine dédié, le mode d'analyse d'haleine comprenant la commande des réglages des parties du système CVC (12) pour qu'elles soient dans des positions qui améliorent les possibilités que l'analyseur d'haleine (11) effectue la détermination de la concentration de la substance cible, dans lequel le mode d'analyse d'haleine comprend au moins une période pendant laquelle le système CVC (12) est agencé pour être dans une position fermée, la position fermée ne permettant pas l'échange de l'air dans le compartiment avec l'air extérieur et provoquant ainsi une accumulation de concentration de substance cible dans le compartiment et au moins pendant cette période, l'unité de commande centrale (13) fait en sorte que l'analyseur d'haleine (11) analyse l'air du compartiment, **caractérisé en ce que**
le système CVC (12) est agencé pour passer rapidement à une position ouverte et dans lequel au moins pendant la transition de la position fermée à la position ouverte, l'unité de commande centrale (13) fait en sorte que l'analyseur d'haleine (11) analyse l'air du compartiment.

2. Système d'analyse d'haleine et de CVC combinés montés sur véhicule (10) selon la revendication 1, dans lequel le changement de position rapide est conçu pour être effectué après la période en position fermée.

3. Système d'analyse d'haleine et de CVC combinés montés sur véhicule (10) selon l'une quelconque des revendications précédentes, dans lequel l'unité de commande centrale (13) est agencée pour régler ou maintenir le système CVC (12) dans une première position aspirant l'air de l'entrée extérieure pendant première période, régler le système CVC (12) en position fermée pendant une seconde période ultérieure, et ramener le système CVC (12) à la première position après la seconde période aspirant l'air de l'entrée extérieure et provoquant une purge de l'air dans le compartiment ; et activer l'analyseur d'haleine (11) au moins pendant la seconde période.

4. Système d'analyse d'haleine et de CVC combinés montés sur véhicule (10) selon la revendication 3, dans lequel l'unité de commande centrale (13) est agencée pour activer l'analyseur d'haleine (11) au moins également pendant la première période, dans lequel l'analyseur d'haleine (11) est agencé pour mesurer une ligne de base.

5. Système d'analyse d'haleine et de CVC combinés montés sur véhicule (10) selon l'une quelconque des revendications précédentes, comprenant en outre des moyens de détection pour détecter la présence d'un conducteur à l'intérieur du compartiment et dans lequel l'unité de commande centrale (13) est agencée pour placer le système CVC (12) dans un mode d'analyse d'haleine dédié sur la base de l'entrée provenant des moyens de détection de présence du conducteur.

6. Système d'analyse d'haleine et de CVC combinés montés sur véhicule (10) selon la revendication 5, dans lequel l'unité de commande centrale (13) est agencée pour changer la position de la vanne et/ou activer l'analyseur d'haleine (11) sur la base de l'entrée provenant des moyens de détection de présence du conducteur.

7. Système d'analyse d'haleine et de CVC combinés montés sur véhicule (10) selon les revendications 3 et 6, dans lequel l'unité de commande centrale (13) est agencée pour régler ou maintenir la vanne dans la première position aspirant l'air de l'entrée extérieure pendant une première période après une détection d'un conducteur.

8. Système d'analyse d'haleine et de CVC combinés montés sur véhicule (10) selon la revendication 4, dans lequel l'unité de commande centrale (13) est agencée pour activer l'analyseur d'haleine (11) pendant la première période, dans lequel l'analyseur d'haleine (11) est agencé pour mesurer une ligne de base, et l'unité de commande centrale (13) est agencée pour activer l'analyseur d'haleine (11) pendant la seconde période, dans lequel l'analyseur d'haleine (11) est conçu pour déterminer la concentration de la substance cible dans le compartiment pendant la recirculation, et dans lequel l'analyseur d'haleine (11) est agencé pour déterminer la concentration de la substance cible dans le compartiment pendant la purge d'air.

9. Système d'analyse d'haleine et de CVC combinés montés sur véhicule (10) selon l'une quelconque des revendications précédentes, dans lequel l'analyseur d'haleine (11) est intégré dans le système CVC (12) de sorte que l'entrée intérieure est une entrée vers le système CVC (12) et l'analyseur d'haleine.

10. Système d'analyse d'haleine et de CVC combinés montés sur véhicule (10) selon la revendication 9, dans lequel l'analyseur d'haleine (11) est agencé en aval du ventilateur (18) et de la vanne du système CVC.

11. Système d'analyse d'haleine et de CVC combinés montés sur véhicule (10) selon l'une quelconque des revendications précédentes, dans lequel le système CVC (12) est, dans un mode de fonctionnement spécial, agencé pour aspirer l'air vers l'arrière du compartiment via la sortie d'air intérieur (16) et faire passer l'air vers l'analyseur d'haleine.

12. Système d'analyse d'haleine et de CVC combinés montés sur véhicule (10) selon l'une quelconque des revendications précédentes, dans lequel l'analyseur d'haleine (11) est agencé pour mesurer une concentration de substance cible et une concentration de gaz traceur.

13. Procédé de détermination d'une substance cible à l'aide d'un système d'analyse d'haleine et de CVC combinés montés sur véhicule (10) agissant sur un compartiment du véhicule, dans lequel le système d'analyse d'haleine et de CVC combinés (10) comprend un analyseur d'haleine (11) agencé pour analyser l'air du compartiment et pour déterminer une substance cible et un gaz traceur dans un échantillon d'air, un système CVC (12) comprenant des parties du système CVC pour commander le débit d'air dans le système CVC (12) et dans le compartiment, les parties du système CVC comprenant au moins une entrée d'air extérieur (15), au moins une entrée d'air intérieur (17) et au moins une sortie d'air intérieur (16), au moins un ventilateur (18) et au moins une vanne, le système CVC (12) étant agencé pour avoir une pluralité de modes de fonctionnement représentant différentes opérations de ventilation, de chauffage et de refroidissement, et une unité de commande centrale (13) agencée pour commander l'analyseur d'haleine (11) et le système CVC, le procédé comprenant
le remplacement d'un mode actuel du système CVC (12) pour placer le système CVC (12) dans un mode d'analyse d'haleine dédié, le mode d'analyse d'haleine comprenant la commande d'au moins l'une des parties du système CVC pour qu'elles soient dans une position qui facilite la réalisation par l'analyseur d'haleine (11) d'une détermination de substance cible, et dans lequel le mode d'analyse d'haleine comprend au moins une période pendant laquelle le système CVC (12) est dans une position fermée, provoquant ainsi une accumulation de concentration de substance cible et de concentration de gaz traceur dans le compartiment et au moins pendant cette période, l'unité de commande centrale (13) fait en sorte que l'analyseur d'haleine (11) analyse l'air du compartiment, **caractérisé en ce que**
l'analyseur d'haleine (11) est actif et détermine la concentration de la substance cible lors du passage du système CVC (12) de la position ouverte à la position fermée ou de la position fermée à la position ouverte.

14. Procédé selon la revendication 13, comprenant en outre les étapes de
- détection de la présence d'un conducteur ; et
- de placement du système CVC (12) dans un mode d'analyse d'haleine dédié sur la base de la détection d'un conducteur.

15. Procédé selon la revendication 13, dans lequel le placement du système CVC (12) dans un mode d'analyse d'haleine dédié comprend le changement de position de la vanne et/ou l'activation de l'analyseur d'haleine.

16. Procédé selon la revendication 13, comprenant les étapes de
- lors d'une première période où le système CVC (12) est agencé pour être réglé ou maintenu dans une première position, l'aspiration d'air de l'entrée extérieure,
- lors d'une seconde période ultérieure, le réglage du système CVC (12) en position fermée, l'aspiration de l'air de l'entrée d'air intérieur (17), provoquant une recirculation de l'air dans le compartiment et l'activation de l'analyseur d'haleine, et
- le retour du système CVC (12) à la première position après la seconde période de temps, l'aspiration d'air de l'entrée externe et la provocation d'une purge de l'air dans le compartiment.

17. Procédé selon la revendication 16, dans lequel l'analyseur d'haleine (11) est activé pendant la première période, dans lequel l'analyseur d'haleine (11) mesure une ligne de base.

18. Procédé selon la revendication 17, comprenant en outre la mesure au moyen de l'analyseur d'haleine (11) pendant l'étape de purge d'air.

19. Procédé selon l'une quelconque des revendications 13 à 18, comprenant en outre, au moyen du système CVC (12), l'aspiration d'air vers l'arrière du compartiment via la sortie d'air intérieur (16) et le passage de l'air vers l'analyseur d'haleine, et le fait d'ordonner au conducteur de diriger son souffle vers la sortie d'air intérieur (16).

20. Procédé selon l'une quelconque des revendications 14 à 19, comprenant en outre, au moyen du système CVC (12), l'aspiration d'air vers l'arrière du compartiment via une entrée dédiée et le passage de l'air vers l'analyseur d'haleine, et le fait d'ordonner au conducteur de diriger son souffle vers l'entrée dédiée.
